# EUROPEAN PATENT APPLICATION

(11) **EP 4 588 958 A1**
(43) Date of publication of application: **23.07.2025**
(21) Application number: 25151909.6
(22) Date of filing: 15.01.2025
(51) Int. Cl.: C08J 3/075, B01J 20/28, C08J 9/28

(54) **METHOD OF PRODUCING MESO-MACROPOROUS HYDROGEL STRUCTURE, HYDROGEL STRUCTURE PRODUCED THEREBY, AND METHOD OF ISOLATING EXOSOMES USING THE SAME**

(30) Priority: 18.01.2024 KR 20240007788
(71) Applicant: Korea Institute of Science and Technology, Seoul 02792 (KR); Korea University Research and Business Foundation, Seoul 02841 (KR)
(72) Inventor: CHOI, Nakwon, 02792 Seoul (KR); KANG, Ji Yoon, 02792 Seoul (KR); KIM, Junbeom, 02075 Seoul (KR); BONG, Ki Wan, 02841 Seoul (KR)
(74) Representative: advotec.

(57) **Abstract**

Provided are a method of producing a meso-macroporous hydrogel structure, a hydrogel structure produced thereby, and a method of isolating exosomes using the same. The meso-macroporous hydrogel structure of the present invention is manufactured from PEGDA, distilled water, and a photoinitiator. Ice crystals grown by freezing at low temperatures act as a porogen, resulting in a porous structure.

## Description

### CROSS-REFERENCE TO RELATED APPLICATION

This application claims the benefit of Korean Patent Application No. 10-2024-0007788 filed on January 18, 2024, in the Korean Intellectual Property Office, the entire disclosure of which is incorporated herein by reference for all purposes.

### BACKGROUND

### 1. Field

One or more embodiments relate to a method of producing a meso-macroporous hydrogel structure, a hydrogel structure produced thereby, and a method of isolating exosomes using the same.

### 2. Description of Related Art

Exosomes are known as external vesicles, extracellular vesicles (EVs), secretive microvesicles, etc., and nano-sized vesicles which are secreted from cells in an external environment for intracellular exchange (Thery C et al., J Extracel Vesic. 2018;7(1): 1535750). The exosomes are known to serve as mediators for intercellular communication, which deliver a functional cargo including various biologically active substances, such as proteins, lipids, nucleic acids, and metabolites. Recently, it has been known that miRNAs in exosomes perform important functions *in vivo* (Yanez-Mo M et al., J Extracel Vesic. 2015;4:27066, Colombo M et al., Ann Rev Cell Dev Biol. 2014;30:255-89, Valadi H et al., Nat Cell Biol. 2007;9(6):654-9). The exosomes are found even in various body fluids and used even for diagnosis of diseases, and can be relatively and stably produced and stored to be developed as diagnostic and therapeutic agents. Despite the clinical utility of these exosomes, conventional methods for isolating exosomes do not satisfy both accessibility and efficiency in isolating EVs and are highly equipment-dependent. The conventional methods require preparation and are labor-intensive, and thus, there is a need for a method of isolating EVs from biofluids that is equipment-free, simple, and effective.

### SUMMARY

One or more embodiments provide a method of producing a meso-macroporous hydrogel structure.

One or more embodiments provide a meso-macroporous hydrogel structure produced by the method.

One or more embodiments provide a method of isolating exosomes using a meso-macroporous hydrogel structure.

However, technical goals to be achieved are not limited to those described above, and other goals not mentioned above can be clearly understood by one of ordinary skill in the art from the following description.

According to an aspect, there is provided a method of producing a meso-macroporous hydrogel structure, including steps a) providing a precursor solution including 10 to 20% [v/v] of poly(ethylene glycol) diacrylate (PEGDA), 78 to 88% [v/v] of distilled water, and 2% [v/v] of a photoinitiator, b) freezing the precursor solution at a low temperature to grow crystals, c) forming a gelled hydrogel by photocrosslinking the frozen precursor solution with ultraviolet irradiation, and d) thawing and washing the hydrogel at room temperature.

In step c), ultraviolet rays may have a wavelength of 365 nm.

In step c), the ultraviolet irradiation may be performed for at least 2 minutes at a UV power density of 2.5 mW/cm².

In step a), PEG may have a number average molecular weight (Mn) of 500 to 1,000.

In step a), the photoinitiator may be 2-hydroxy-2-methylpropiophenone.

Step b) may include freezing the mixture at -80°C for at least 10 minutes.

According to another aspect, there is provided a meso-macroporous hydrogel structure produced by the above-described method.

Pores of the meso-macroporous hydrogel structure may have a diameter of 50 to 400 nm.

The meso-macroporous hydrogel structure may be used for an isolation of exosomes.

According to another aspect, there is provided a method of isolating exosomes using a meso-macroporous hydrogel structure, including steps a) mixing a biological sample of a subject with an exosome precipitation buffer having an NaCl concentration of 1.5 M or higher, b) adding the meso-macroporous hydrogel structure of claim 6 to the mixture of the exosome precipitation buffer and the biological sample and precipitating exosomes inside a gel, and c) adding the hydrogel structure in which the precipitation is completed to an exosome elution buffer having an NaCl concentration of less than 1.5 M to elute the exosomes.

Additional aspects of embodiments will be set forth in part in the description which follows and, in part, will be apparent from the description, or may be learned by practice of the disclosure.

According to embodiments, using a method of producing a meso-macroporous hydrogel structure, it is possible to obtain a porous hydrogel structure having a desired pore size by controlling a freeze-drying time, and to enable mass production at a low cost.

In addition, a meso-macroporous hydrogel structure produced by the method according to an embodiment may have a pore size of 400 nm or less, suitable for an isolation of exosomes, and have continuous performance and stability.

Furthermore, a method of isolating exosomes using the meso-macroporous hydrogel structure according to an embodiment may not require preprocessing of biological samples and may be relatively simple, and thus, may be usefully used for early diagnosis of diseases, prediction of prognosis, and the like.

The effects of the present disclosure are not limited to the aforementioned effects, and other effects, which are not mentioned above, will be clearly appreciated by one of ordinary skill in the art from the following description.

### BRIEF DESCRIPTION OF THE DRAWINGS

These and/or other aspects, features, and advantages of the invention will become apparent and more readily appreciated from the following description of embodiments, taken in conjunction with the accompanying drawings of which:
FIGS. 1A to 1C illustrate the overview of production of a meso-macroporous hydrogel structure and hydrogel-based extracellular vesicle (EV) isolation;
FIGS. 2A to 2D illustrate characterization of a meso-macroporous hydrogel;
FIGS. 3A to 3G illustrate results of hydrogel-based EV isolation;
FIGS. 4A to 4F illustrate characteristics of produced meso-macroporous hydrogel particles;
FIGS. 5A and 5B illustrate a cyro-photocrosslinking process and recovery volumes;
FIGS. 6A and 6B illustrate a basic principle and characteristics of EV isolation using a meso-macroporous hydrogel structure;
FIGS. 7A to 7D illustrate a retention of meso-macroporous hydrogel particles;
FIGS. 8A to 8E illustrate EV isolation and downstream analysis based on a meso-macroporous hydrogel structure from various biofluids;
FIGS. 9A and 9B illustrate results of EV isolation from a plasma based on a hydrogel;
FIGS. 10A to 10E illustrate characteristic analysis of EVs isolated from a plasma; and

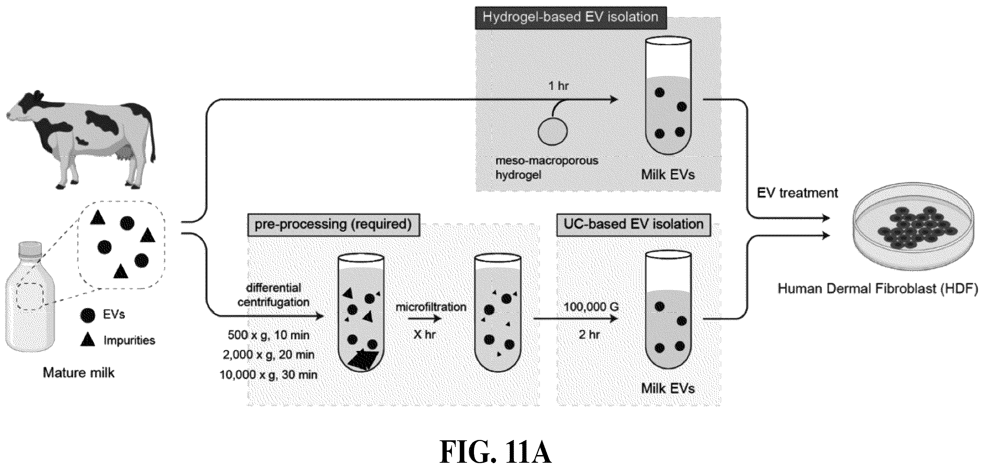

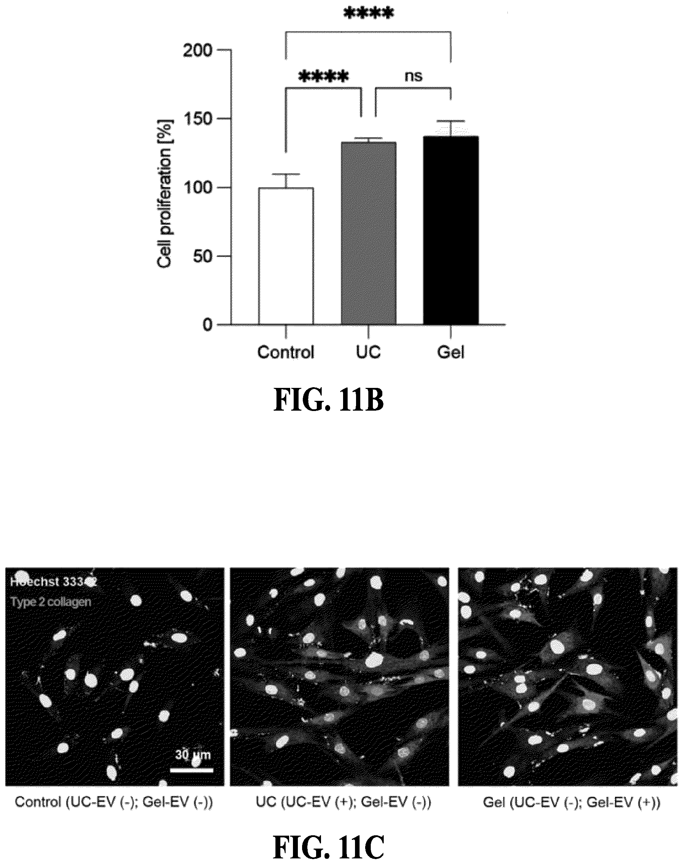

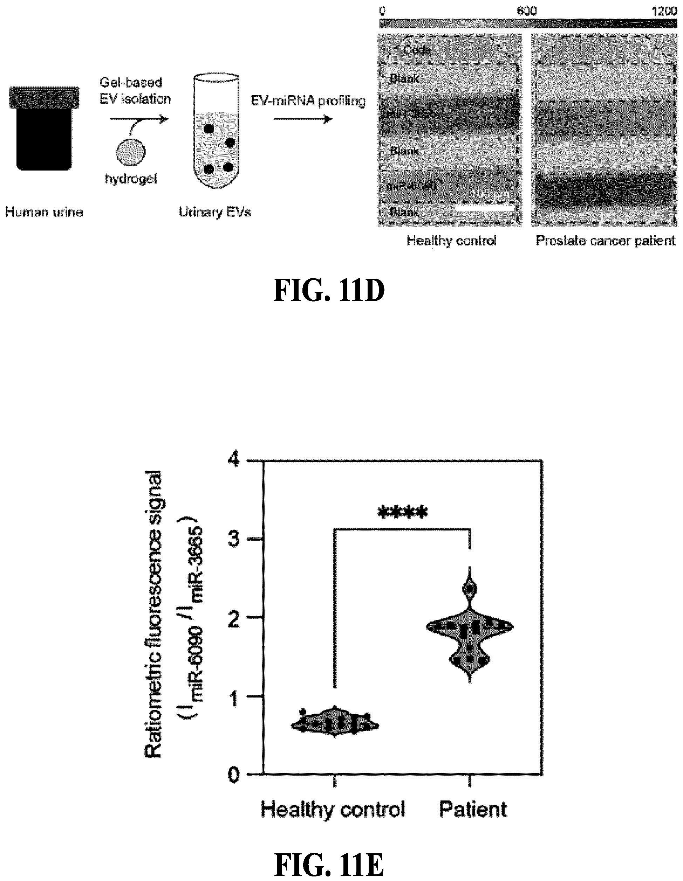

FIGS. 11A to 11E illustrate a schematic diagram of a process of isolating exosomes based on a meso-macroporous hydrogel according to an embodiment.

### DETAILED DESCRIPTION

The present inventors have completed the present disclosure by conceiving that when PEG of a specific composition is frozen for a certain period of time before photocrosslinking and gelling into a hydrogel, water molecules become ice crystals and act as porogens, and then the photocrosslinked hydrogel has a porous structure. A porous hydrogel of the present disclosure may achieve a desired pore size by controlling the freezing time, and in particular, may be controlled to a size suitable for isolating exosomes, which is an aspect of the present disclosure. Hereinafter, the description thereof is provided.

More specifically, one or more embodiments may provide a method of producing a meso-macroporous hydrogel structure including steps a) providing a precursor solution including 10 to 20% [v/v] poly(ethylene glycol) diacrylate (PEGDA), 78 to 88% [v/v] distilled water, and 2% [v/v] of a photoinitiator, b) freezing the mixture at a low temperature to grow crystals, c) forming a gelled hydrogel by photocrosslinking the frozen precursor with ultraviolet irradiation, and d) thawing and washing the hydrogel at room temperature.

As used herein, the term "meso-macroporous" refers to having a pore size between "macroporous" and "microporous," which are terms commonly used in the art, and desirably, may be a size suitable for filtering exosomes or extracellular vesicles (EVs), and a non-limiting numerical range thereof may be a pore diameter of 50 to 400 nm.

As used herein, the term "exosome" refers to a membrane vesicle contained in a human cell or secreted out of the human cell, which contains various substances exhibiting biological activity, such as lipids, nucleic acids, proteins, and metabolites therein. The exosomes may be any one or more selected from the group consisting of EVs, microvesicles, extracellular vesicles, microvesicles, or microparticles, but are not limited thereto, and in this specification, the terms "EV" and "exosome" may be used interchangeably with the same meaning.

In an aspect, an ultraviolet wavelength in step c) may vary depending on a type of photoinitiator, but desirably may have a wavelength of 365 nm, and in this case, the photoinitiator may be 2-hydroxy-2-methylpropiophenone.

According to one aspect, the ultraviolet irradiation in step c) may be performed for at least 2 minutes at a UV power density of 2.5 mW/cm².

According to one aspect, in step a), PEG may have a number average molecular weight (Mn) of 500 to 1,000, desirably 700.

According to one aspect, step b) may include freezing the mixture at -80°C for at least 10 minutes. Under the freezing condition, ice crystals may grow to a size suitable for exosome isolation.

According to another embodiment, a meso-macroporous hydrogel structure produced by the above-described method may be provided.

According to one aspect, pores of the meso-macroporous hydrogel structure may have a diameter of 50 to 400 nm.

According to one aspect, the meso-macroporous hydrogel structure may be used for an isolation of exosomes.

According to yet another embodiment, a method of isolating exosomes using a meso-macroporous hydrogel structure including steps a) mixing a biological sample of a subject with an exosome precipitation buffer having an NaCl concentration of 1.5 M or higher, b) adding the meso-macroporous hydrogel structure to the mixture of the exosome precipitation buffer and the biological sample and precipitating exosomes inside a gel, and c) adding the hydrogel structure in which the precipitation is completed to an exosome elution buffer having an NaCl concentration of less than 1.5 M to elute the exosomes.

The exosome precipitation buffer in step a) may be a buffer having an NaCl concentration of 1.5 M or more and a HEPES concentration of 20 mM or more, and the concentration after mixing with the biological sample may be at least 1.5 M NaCl and 20 mM HEPES. The exosomes may be precipitated within pores of a porous hydrogel structure under a relatively high concentration of an exosome precipitation buffer, and then may be eluted from the structure under a relatively low concentration of an exosome elution buffer in step c). The porous hydrogel structure according to an embodiment may effectively isolate exosomes having a less than pore size through precipitation and elution according to a difference in salt concentration as described above. The exosome elution buffer of step c) may have a NaCl concentration of 1.5 M, but desirably may have an NaCl concentration of 0 M.

The "subject" is not limited to any mammal such as livestock or humans that requires diagnosis using exosomes, but may be desirably humans, and most desirably humans that require prevention, treatment, and/or diagnosis of prostate cancer.

The "biological sample" is not limited to anything from which exosomes of the subject may be extracted, and non-limiting examples thereof may be whole blood, plasma, urine, saliva, semen, nasal swab, nasopharyngeal swab, and prostate biopsy sample.

The terms used in the embodiments are used for the purpose of description only, and should not be construed to be limited. A singular expression includes a plural expression unless the context clearly indicates otherwise. In this specification, it should be understood that the term "including" or "having" indicates that a feature, a number, a step, an operation, a component, a part or the combination thereof described in the specification is present, but does not exclude a possibility of presence or addition of one or more other features, numbers, steps, operations, components, parts or combinations thereof, in advance.

Unless otherwise contrarily defined, all terms used herein including technological or scientific terms have the same meanings as those generally understood by a person with ordinary skill in the art to which embodiments pertain. Terms which are defined in a generally used dictionary should be interpreted to have the same meaning as the meaning in the context of the related art, and are not interpreted as ideal or excessively formal meanings unless otherwise defined in the present disclosure.

The present disclosure may have various modifications and various Examples, and specific Examples will be hereinafter illustrated in the drawings and described in detail in the detailed description. However, the present disclosure is not limited to specific embodiments, and it should be understood that the present disclosure covers all the modifications, equivalents and replacements within the idea and technical scope of the present disclosure. In describing the present disclosure, when it is determined that a detailed description of related known arts may obscure the gist of the present disclosure, the detailed description will be omitted.

### Example 1. Experimental Method

### Example 1-1. Preparation of Meso-Macroporous PEGDA Hydrogel Particles

First, a PDMS (Sylgard 184, Dow Corning) mold containing a series of wells was prepared. The size of each PDMS well had a diameter of 5 mm and a depth of 2 mm. A degassed mixture of PDMS and curing agent was poured at a weight ratio of 10 : 1 in a master made of milled duralumin or a 3D printer (M50-405, CADworks3D) photopolymer resin (Microfluidic Mastermold Resin, CADworks3D). The PDMS mixture was cured at 58°C for 3 hours, and then a PDMS replica was peeled off.

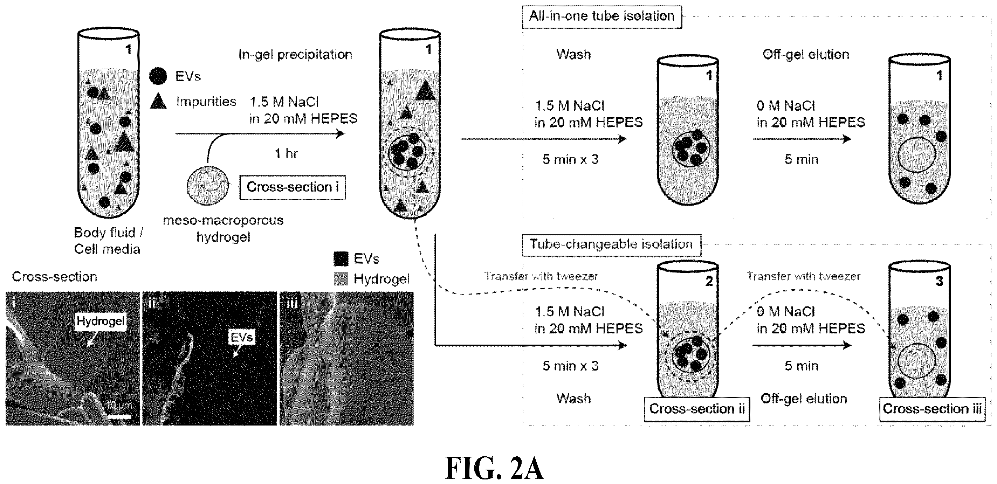

Next, a precursor solution including 10% [v/v] PEG700DA (Cat. # 455008, Sigma-Aldrich), 88% [v/v] DIwater, and 2% [v/v] 2-hydroxy-2-methylpropiophenone (Irgacure 1173; Cat. # 405655; Sigma-Aldrich) as a photoinitiator was prepared. For experimental data presented in FIG. 2A, a volume composition of PEG700DA was changed while maintaining the sum of the two compositions at 98% [v/v] using DI water.

Thereafter, 40 µL of a precursor solution was applied to each PDMS well. To remove the potential effect of freezing of the precursor solution due to room temperature change, the precursor-loaded mold was placed in a refrigerator at 4°C for 10 minutes. This pre-step was optional and did not affect the quality control of the preparation of meso-macroporous PEGDA hydrogel particles. Thereafter, the precursor-loaded mold was frozen at -80°C for 10 minutes. After this freezing step, the frozen mold was immediately transferred to a UV chamber (MT-GJ09, Minuta) and the frozen precursor solution was photocrosslinked for 2 minutes at a UV power density of 2.5 mW/cm² (i.e., UV energy density of 300 mJ/cm²). Finally, the crosslinked PEGDA-loaded mold was rinsed with excess DI water for about 1 minute to thaw cryo-photocrosslinked PEGDA particles. Through the thawing step, the photoinitiator and uncrosslinked PEG700DA were washed and free meso-macroporous PEGDA particles were harvested. Depending on the purpose and application, users may store the meso-macroporous hydrogel particles in a wet state in DI water or in a dry state after lyophilization before use. For lyophilization, the meso-macroporous hydrogel particles were immersed in liquid nitrogen and lyophilized overnight in a lyophilizer (TFD5503; IlShinBioBase).

### Example 1-2. Isolation of EVs using Meso-Macroporous PEGDA Hydrogel Particles

First, a 2× EV precipitation buffer including 3 M NaCl and 40 mM HEPES in DI water was prepared. To prepare an EV isolation medium containing a final concentration of 1.5 M NaCl and 20 mM HEPES, the 2× EV precipitation buffer was mixed with an equal volume of biofluid (human whole blood, plasma, saliva (oral swirl), urine, milk, or ESC culture medium). Second, meso-macroporous PEGDA particles were applied to the EV isolation medium at a dose (4 particles/mL for whole blood and plasma; 1 particle/mL for saliva, urine, and milk; 0.25 particles/mL for ESC culture medium) and then incubated at 4°C for 1 hour with rotation (10 rpm, B008DZ2VUG, FinePCR) indicating in-gel precipitation. Next, the particles were washed three times using a rinse buffer including 1.5 M NaCl and 20 mM HEPES in DI water, and each wash contained a volume five times greater than the initial volume of particles used for in-gel precipitation. Finally, EVs were isolated from the particles using an EV elution buffer including 20 mM HEPES in NaCl-free water and prepared to be used for additional analysis or other experiments. The volume of EV elution buffer was adjusted as needed. It was confirmed that the EVs recovered through the final step did not contain a high concentration of NaCl capable of interfering with downstream analysis or functional studies. For all-in-one tube isolation, the steps were performed in the same tube, and for tube-replaceable isolation, the meso-macroporous hydrogel particles were transferred to a new tube using forceps.

### Example 1-3. EV Isolation by Ultracentrifugation

First, two pre-steps of centrifugation and microfiltration were performed sequentially. Each biofluid was centrifuged sequentially at 500×g for 10 minutes, 2,000×g for 20 minutes, and 10,000×g for 30 minutes using a high-speed centrifuge (1236R; LaboGene). Next, syringe filtration was performed through a 450 nm membrane filter (Millex). Then, the pretreated biofluid was ultracentrifuged at 100,000×g for 120 minutes using a Type 70 Ti rotor (Beckman Coulter). Finally, after ultracentrifugation, the resulting pellets were resuspended in phosphate-buffered saline (PBS). The amount of PBS for resuspension was the same as the amount of EV-rec/Overly buffer for the corresponding meso-macroporous hydrogel-based EV isolation. All centrifugation steps were performed at 4°C.

### Example 1-4. Nanoparticle Tracking Analysis (NTA)

The size distribution of the isolated EVs was identified and quantified using nanoparticle tracking analysis (NTA, NanoSight LM10, Malvern) according to the manufacturer's protocol. That is, to adjust a particle concentration (1.0 × 10⁸ to 2.5 × 10⁹ particles/mL) suitable for analysis, samples (10X dilution for plasma and urine; 1X dilution for saliva and ESC culture medium) were diluted with exosome-depleted PBS (dPBS). Then, 400 µL of the diluted samples were injected into an instrument using a 1 mL syringe. Imaging settings, including camera level 12, analysis gain 4, etc. were manually set according to the manufacturer's instructions. Thereafter, the acquired data were controlled again to estimate the EV concentration before dilution.

### Example 1-5. Bradford Assay for Purity Evaluation

A protein concentration of an EV isolation solution was estimated by Bradford assay (protein analysis dye reagent, Bio-Rad). 5 µL of an EV recovery solution was mixed with 250 µL of a Bradford reagent, and the mixture was dispensed into a 96-well microplate (Corning). Then, after incubation for 10 minutes at room temperature, the absorbance of the mixture was measured at 595 nm using a microplate reader (Synergy HTX, BioTek).

### Example 1-6. Western and Dot Blot

For Western blot, total proteins of an EV isolation line were first extracted using a RIPA cell lysis buffer (1×; GenDEPOT). After loading the extracted proteins for sodium dodecyl sulfate-polyacrylamide gel electrophoresis (SDS-PAGE), the gel was transferred to a NitroPure Nitrocellulose transfer membrane (GenDEPOT) for blotting. The following primary and secondary antibodies conjugated with horseradish peroxidase (HRP) were used to immunolabel a target protein: anti-CD63 antibody (ab8219; Abcam), anti-TSG101 antibody (ab125011; Abcam), anti-calnexin antibody (ab22595; Abcam), goat anti-mouse IgG H&L (ab205719; Abcam), and goat anti-rabbit IgG H&L (ab205718; Abcam). Luminescence corresponding to the expression of the target protein was emitted using SuperSignal^{™} West Femto Maximum Sensitivity Substrate (Thermo Scientific) and imaged with a Davinch-Chemi^{™} chemiluminescence imaging system (Cas400MF; Davinch-K). For dot blot, immunolabeling and imaging were performed in the same manner as for Western blot without sample loading and transfer.

### Example 1-7. Diffusion of Fluorescent Indicator into PEGDA Particles

In order to indirectly investigate the porosity of meso-macroporous and microporous PEGDA particles, one-dimensional radial diffusion of various fluorescent indicators was performed on a cylindrical PEGDA post with impermeable upper and lower boundaries. Fluorescent indicators were fluorescein (347.3 Da; ~1 nm; #201626; Sigma-Aldrich), FITC-dextran (10 kDa; 4.72 nm; FD10S; Sigma-Aldrich), green fluorescent polystyrene nanobeads (100 and 200 nm; DNG-L015 and -L019; CD bioparticles), and silica nanobeads (450 nm).

For meso-macroporous and microporous hydrogels, cylindrical PEGDA posts with a diameter of 900 µm were fabricated by photocrosslinking the precursor solution between two 1 mm-thick glass slides spaced apart by a double-sided tape (to 250 µm thick), regardless of pre-freezing. A field diaphragm, which served as a photomask for UV crosslinking, was adjusted on an inverted microscope (Axio Observer.A1, Zeiss) to set the diameter. After low-temperature or conventional photocrosslinking, the PEGDA posts were rinsed with DI water to remove uncrosslinked PEGDA and photoinitiator.

Each fluorescent indicator was placed in a gap between the glass slides and diffused for 1 hour while sealing the gap with a parafilm to prevent evaporation. To acquire 16-bit black-and-white fluorescence images on the inverted microscope, a light-emitting diode (LED) lamp (SOLA SM 2, Lumencor Light Engine) was used as a source, a filter cube set (89101 x, 89101 m, and 89100 bs; Chroma Technology) was used, and a scientific complementary metal oxide semiconductor (sCMOS) camera (optiMOS, QImaging) with an exposure time of 10 ms was used.

### Example 1-8. Scanning Electron Microscope (SEM)

For SEM of meso-macroporous PEGDA hydrogel particles before and after precipitation in the gel and before and after off-gel elution of EVs, the particles were first lyophilized at each step. Thereafter, SEM (Nova Nano SEM 200, FEI Company) provided by the Advanced Analysis Center of the Korea Institute of Science and Technology (KIST) was performed.

### Example 1-9. Obtainment of Stem Cell Culture Medium

To harvest a stem cell culture medium for EV isolation, 2D culture of an H1 human embryonic stem cell line (H1ESC) was performed in a 6-well plate. The cell line was grown in a feeder-free environment coated with Matrigel and Modified Eagle Medium Nutrient Mixture F-12 at a ratio of 1 : 25, respectively. Stem cell culture was maintained using a feeder and a serum-free maintenance medium mTeSR^{™}1 cGMP for human ESC and induced pluripotent stem cells (iPSC), in a humidified incubator at 37°C and 5% CO₂. Then, when many H1ESC colonies of about 0.5 mm were formed in each well, the maintenance medium was collected within 5 to 7 days. The culture medium was dispensed into 15 mL conical tubes without additional washing and the tubes were stored at -80°C before EV isolation.

### Example 1-10. Obtainment of Saliva Sample

A saliva sample was collected by swirling 12 mL of sterile DI water for 1 minute and spitting it into a sterile conical tube. The oral swirl was centrifuged at 2,000× g for 10 minutes at 4°C to remove a relatively large lump (e.g., food residues) from the mouth, and 10 mL of the upper portion was stored at -80°C before EV isolation.

### Example 1-11. Obtainment of Urine Sample

The study protocol was implemented in accordance with the Institutional Review Board (IRB) approval guidelines (IRB approval number 2017AN0036) of Korea University Anam Hospital. All male subjects were requested to urologists for cancer screening due to elevated prostate-specific antigen (PSA) levels, abnormal findings on digital rectal examination (DRE), or hypoechoic lesions of the prostate on transrectal ultrasound. Urine from each subject was collected on the first day of hospitalization for prostate biopsy after DRE. All the subjects underwent transrectal ultrasound-guided prostate biopsy and were classified as prostate cancer patients or healthy controls after pathological examination of specimens. Patients with urothelial carcinoma or other malignant diseases were excluded from the patient group. For urine samples in both groups, 5 mL of urine was pre-centrifuged at 2,000× g for 10 minutes at 4°C. Then, the upper layer (1.8 mL) was dispensed into cryovials and stored at -80°C. The urine biospecimens and data used in this study were provided by the Korea University Anam Hospital Biobank (KUAH2017-02).

### Example 1-12. Urinary EV-miRNA Profiling using Hydrogel-Based Hybridization Chain Reaction (HCR)

Urinary EV-miRNA profiling was performed using hydrogel-based HCR according to a previous study. First, total RNA was extracted from EV isolates in the gel using TRIzol (Invitrogen) according to the manufacturer's instructions. Next, 10 µL of urine EV-RNA was mixed with a suspension of the conformationally encoded internal PEGDA microparticles to initiate the multiplex detection of hsa-miR-3665 and -6090. Thereafter, the fluorescence signal within each probe region of internal plex microparticles was amplified by adding an initiator and biotinylated hairpins to allow HCR. 16-bit black-and-white fluorescence images of microparticles labeled with streptavidin-phycoerythrin (SA-PE) were obtained on an inverted microscope (Axio Observer.A1; Zeiss) using an LED light source (SOLA SM 2; Lumencor Light Engine), a filter cube set (89101 x, 89101 m, and 89100 bs; Chroma Technology), and an sCMOS camera (optiMOS; QImaging). The ratiometric intensities between healthy controls and prostate cancer patients were analyzed as previously reported.

### Example 1-13. Statistical Analysis

All statistical analyses were performed in Prism (GraphPad software). ANOVA was used to compare the means of two or more samples or groups, and a t-test was used to compare the means between two samples. Parametric unpaired t-test and one-way ANOVA with a Dunnett's multiple comparison test were selected to compare data. ns, *, ***, and **** indicated P > 0.05, < 0.05, < 0.001, and < 0.0001, respectively, and the calculated P values were also specified.

### Example 2. Preparation of Meso-Macroporous Hydrogel and Hydrogel-Based EV Isolation

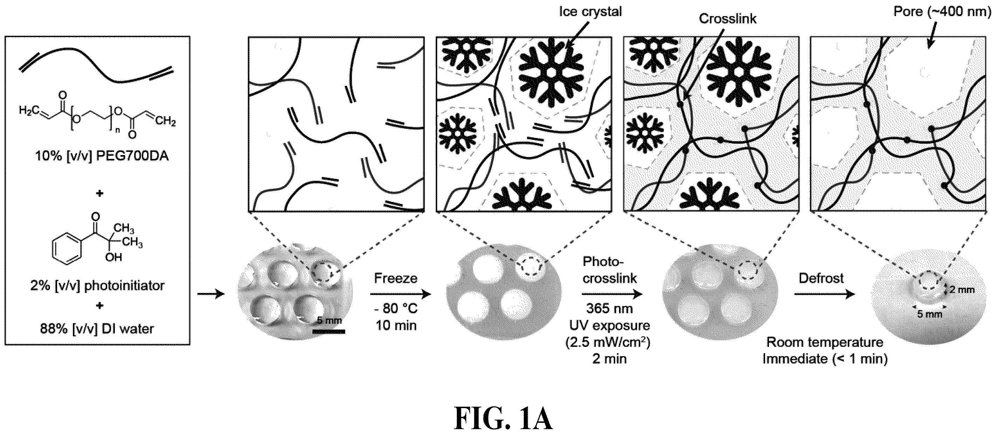

Polyethylene glycol (PEG) hydrogel particles were utilized as a tractable gel medium to spatially confine EVs by precipitation. Meanwhile, liquid PEG served as a precipitant for EVs and bacteriophages. To prepare meso-macroporous hydrogel particles (e.g., 5 mm in diameter and 2 mm in thickness), an aqueous PEGDA precursor solution was photocrosslinked at low temperature (FIG. 1A). Among commercially available products, PEGDA with Mn 700 (PEG700DA) was selected due to the highest Mn (number-average molecular weight) and liquid phase at room temperature and economic feasibility. In contrast, PEGDA with Mn 1,000 or higher existed in a solid state and had a unit price that was 500 times or higher (Table 1).

**[Table 1]**

| Material | Catalog# | Amount | Price [USD] | Price/amount ratio [USD/mL or g] |
|---|---|---|---|---|
| PEG700DA | 455008-500ML | 500 mL | 138.24 | 0.28 |
| Irgacure 1173 | 405655-250ML | 250 mL | 221.73 | 0.89 |
| PEG1000DA | 729086-1g | 1 g | 166.89 | 166.89 |
| Material | Composition | Price per Volume [USD/mL] | Particle volume | Price per particle [USD/particle] |
| Meso-macroporous hydrogel particle | PEG700DA 10% [v/v] + Irgacure 1173 2% [v/v] | 0.045 | 40 µL | 0.0018 |

The precursor solution was rapidly frozen at -80°C for 10 minutes to generate ice crystals as solid-phase porogen. Thereafter, chains of PEG700DA were immediately photocrosslinked to fix 3D embedding of the ice crystals, and ultimately immediately thawed in DI water to form pores having a size of up to 400 nm.

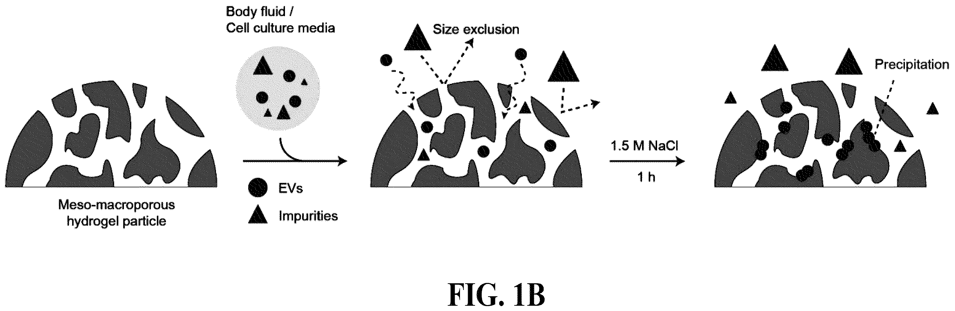

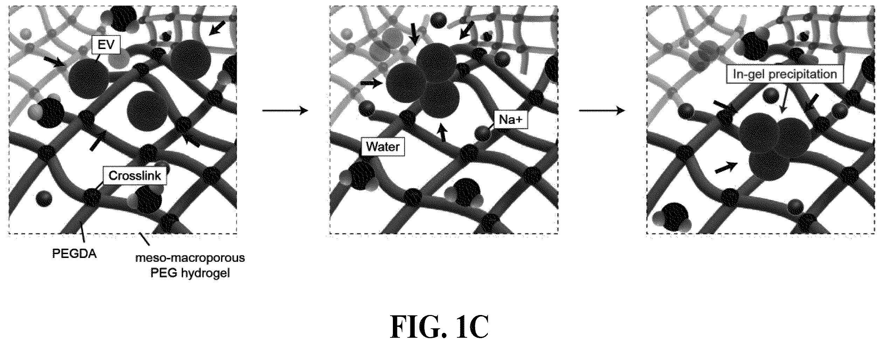

As a result, the meso-macroporous hydrogel particles allowed EV diffusion of less than 400 nm, excluded larger impurities, and then induced in-gel precipitation (FIG. 1B). More specifically, the PEG chains in the gel acted as a precipitant at a high salt (co-precipitant) concentration for equipment-free EV isolation. The basic principle of EV in-gel precipitation similarly follows two components combined with liquid-phase PEG and a salt phenomenon (FIG. 1C). Primarily, the coexistence of the precipitant (PEG) and the co-precipitant (salt) induced only the EV precipitation. During rapid pre-freezing, when ice crystals were formed, PEGDA chains were reorganized to form a locally compact network after low-temperature photocrosslinking (FIG. 1A). In particular, these PEG chains fixed between the crosslinks remained almost immobile. Therefore, transport of EVs into the meso-macropores was equivalent to the local encompassment of EVs by gel-phase PEG chains (left side of FIG. 1C), similar to a situation where EVs were more closely bound to each other by a steric shielding effect of mobile liquid-phase PEG chains. At the same time, a high concentration of Na+ was replaced with surrounding water molecules substantially present near hydrophilic PEG, thereby reducing a repulsive interaction between EVs and making the inter-EV attractive force more favorable (middle side of FIG. 1C). As a result, the binding effect of each component induced the aggregation and precipitation of EVs in the meso-macroporous hydrogel particles (right side of FIG. 1C). Another powerful advantage of the in-gel precipitation, along with the size exclusion function, is that the residual precipitates are essentially removed after EV recovery.

### Example 3. Hydrogel-based EV Isolation

After applying meso-macroporous hydrogel particles to an EV-containing sample to be isolated at an isolation throughput of 1 purified particle (40 µL) per 0.3 to 4 mL, EVs could be isolated for 80 minutes in three steps without pretreatment (e.g., differential centrifugation or microfiltration) or equipment (see at least 120 minutes for ultracentrifugation): 1) in-gel precipitation for 1 hour using 1.5 M NaCl, 2) washing three times for 5 minutes each, and 3) off-gel elution for 5 minutes using 0 M NaCl (FIG. 2A). All working solutions were maintained at a constant pH of 7.4 using 20 mM N-2-hydroxyethylpiperazine-N-2-ethanesulfonic acid (HEPES). Depending on the application (e.g., temporary suspension or storage for integrated deployment of downstream analysis), either an all-in-one tube (top of FIG. 2A) or a changeable tube (bottom of FIG. 2A) isolation may be selected.

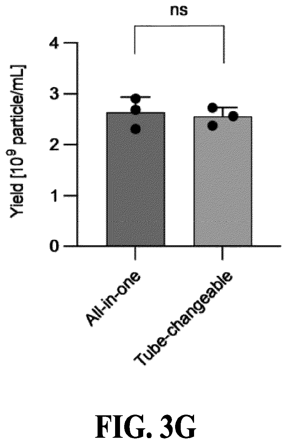

It was confirmed that the all-in-one tube isolation showed almost the same yield (FIG. 3G), but the following experiment was performed using changeable tube isolation to remove unexpected side effects due to residual impurities or salts. In addition, particles having a diameter of 5 mm were intentionally designed so that even non-technical people may transfer the particles to a new tube using tweezers. That is, the centrifugation was unnecessary.

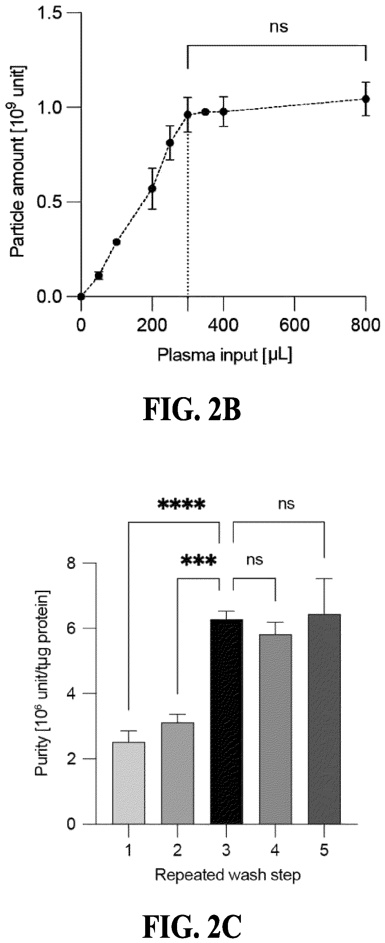

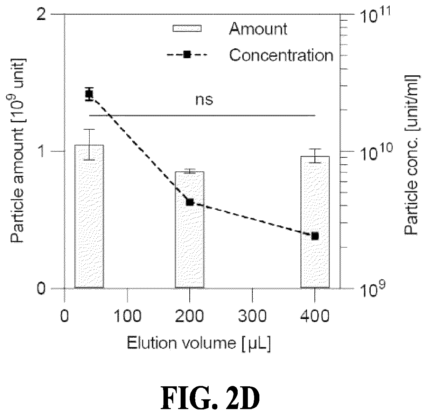

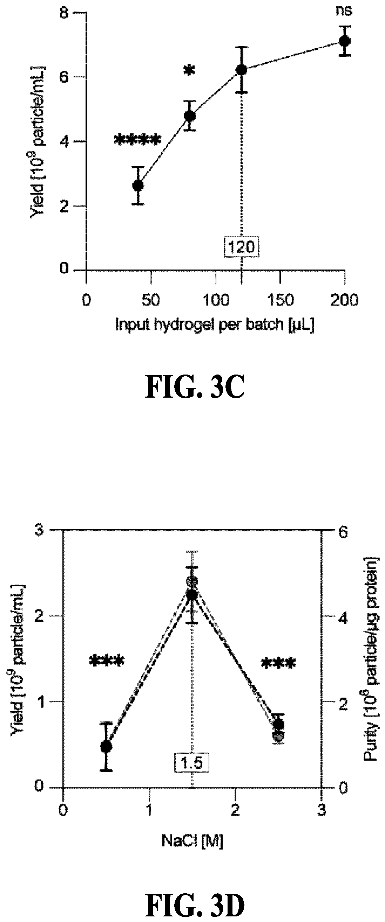

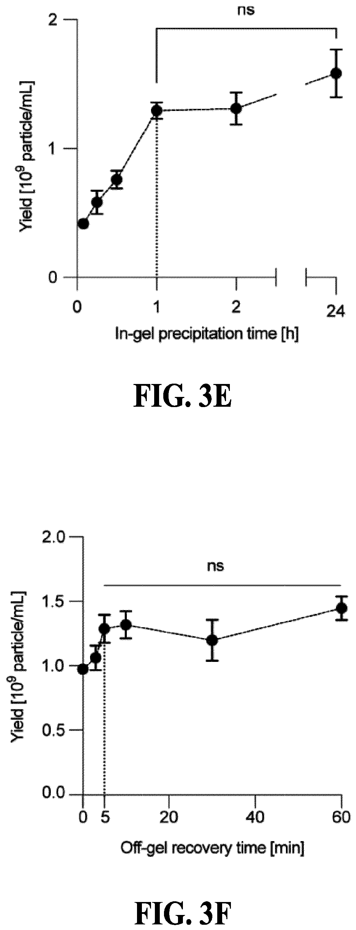

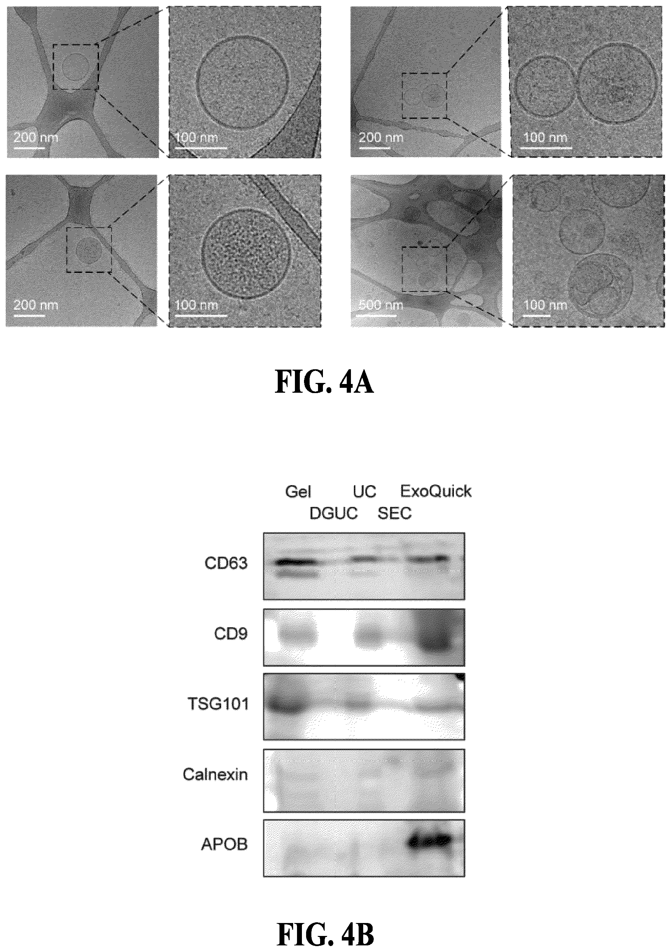

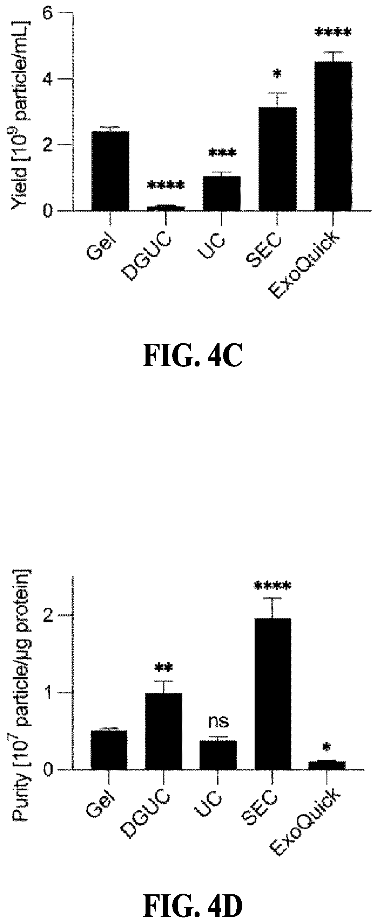

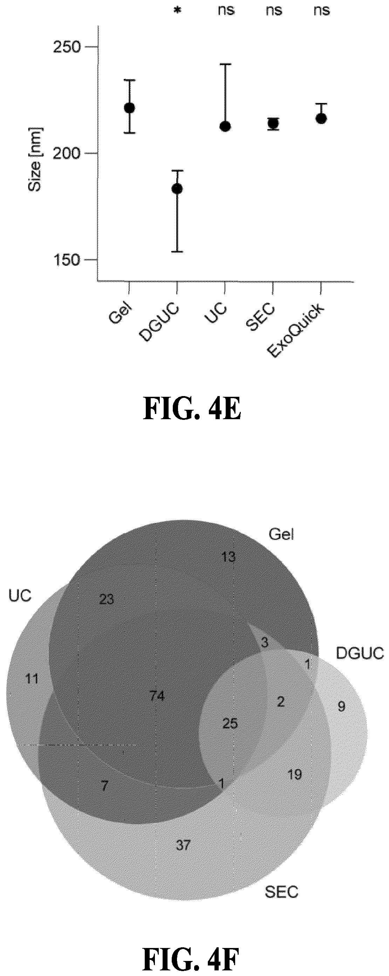

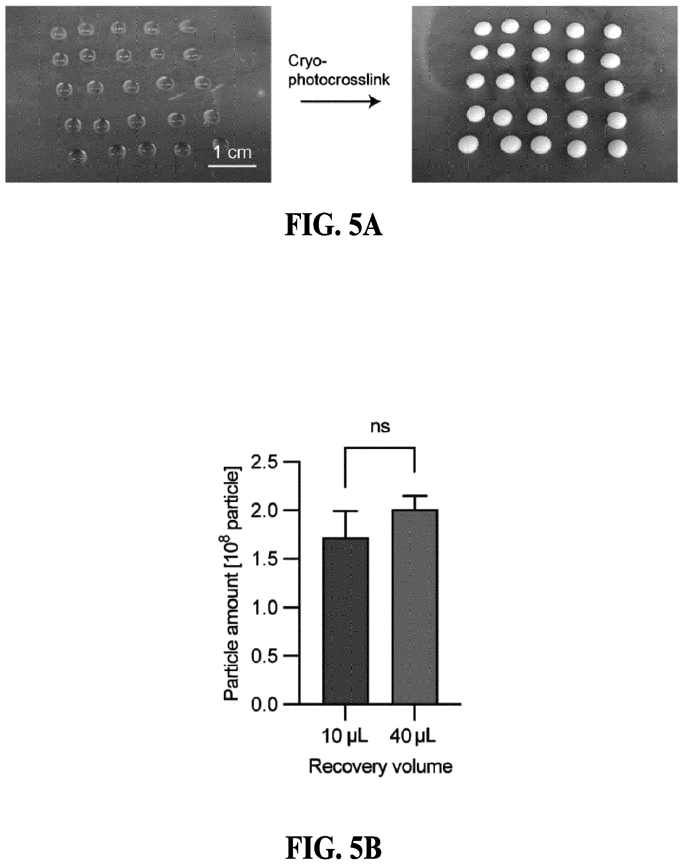

The prepared meso-macroporous hydrogel particles may function as EV-concentrated carriers with a size of mm. Cross-sectional scanning electron microscope (SEM) images (FIG. 2A, i to iii, and FIG. 4A) showed that an initial EV-free hydrogel (FIG. 2A, i) contained EVs after gel-in precipitation and several washes. EVs were successfully eluted under high salt conditions (FIG. 2A, ii) and salt removal (FIG. 2A, iii). Among various operating parameters, an EV isolation capacity (300 µL of human plasma or up to 10⁹ EVs per 40 µL of hydrogel particles, FIG. 2B, FIG. 3C), the number of repetitions of the washing step (three times, FIG. 2C), a possible elution volume (> 40 µL of elution buffer for 40 µL of hydrogel particles, FIG. 2D), optimal precipitation and elution times (1 hour and 5 minutes, respectively, FIGS. 3E and 3F) and a salt concentration (1.5 M NaCl, FIG. 3D) were determined. It was confirmed that a function to easily manipulate the volume and shape of the meso-macroporous hydrogel particles was one of the greatest advantages of the present disclosure when purifying EVs from limited or unlimited sample volumes or when controlling the final concentration of EVs. The final concentration of isolated EVs was precisely controlled by simply changing the amount of elution buffer introduced through the control (FIGS. 2D and 5).

### Example 4. Identification of Characteristics of Meso-Macroporous Hydrogels

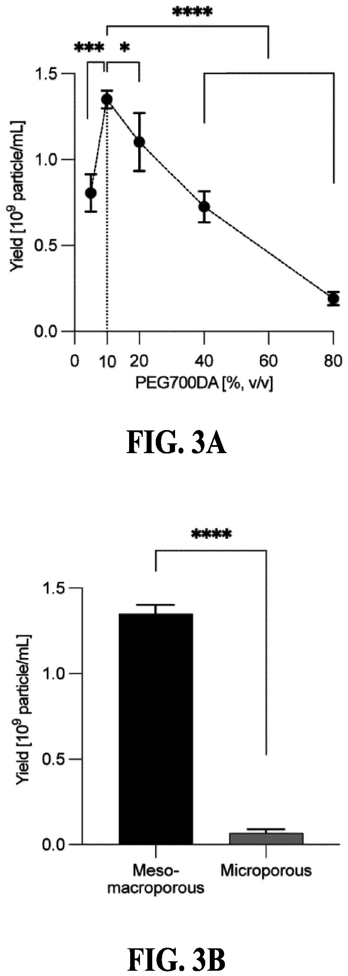

As described above, it was confirmed that the PEG700DA composition was a critical design parameter to achieve meso-macroporosity and successful EV elution. The 10% [v/v] PEG700DA-cryo-photocrosslinked hydrogel showed the highest efficiency for EV isolation from human plasma when the concentration varied from 5 to 80% [v/v] under the same freezing conditions at -80°C for 10 minutes (FIG. 3A). This non-monotonic change was predicted to result from the overlapping competition between PEG-positive and -negative factors. In particular, increased PEG (precipitant) was beneficial for improved EV isolation. At the same time, a denser crosslinked PEG network exhibited undesirable size exclusion due to reduced porosity and tortuosity (i.e., connectivity between pores) that reduced the possibility of EVs migrating into the hydrogel. It has been possible to obtain hydrogel structures by freezing at higher temperatures for longer periods of time to produce ice crystals of a similar size, but empirically, freezing conditions to rapidly fabricate batches over approximately 13 minutes were selected.

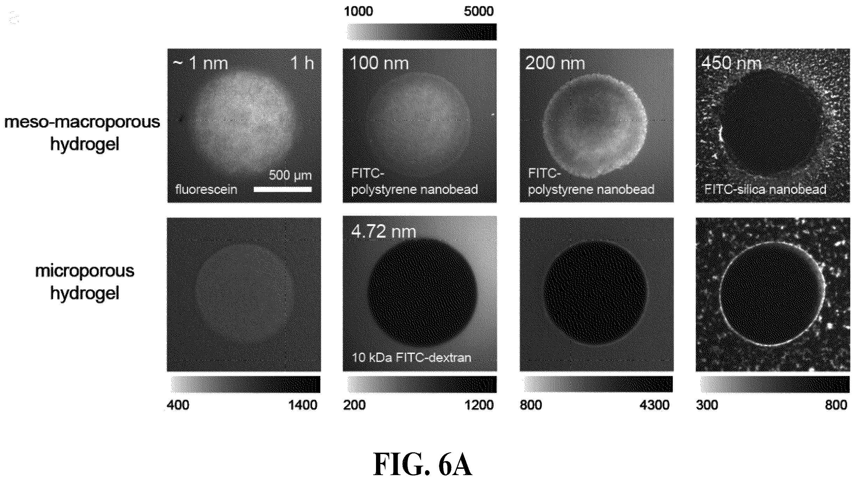

To verify meso-macroporosity, fluorescent indicators of various sizes were diffused into a cryogenic photocross-linked hydrogel post, which were as follows (FIG. 6A): ~ 1 nm (fluorescein; 347.3 Da), 4.72 nm (FITC-dextran; 10 kDa), 100 and 200 nm (green fluorescent polystyrene nanobeads), and 450 nm (FITC-silica nanobeads). While indicators up to 200 nm were well diffused within 1 hour, 450 nm nanobeads were found to be impermeable. In contrast, indicators of 4.72 nm or higher could not penetrate the photocrosslinked microporous hydrogel without pre-freezing. Thus, about 20 times greater efficiency could be achieved in isolating EVs from plasma (FIG. 3B). Morphological characterization of the recovered EVs was performed via cryo-TEM, which enabled visualization of EVs showing lipid bilayer particles of similar sizes to EVs, similarly to other references (FIG. 4A).

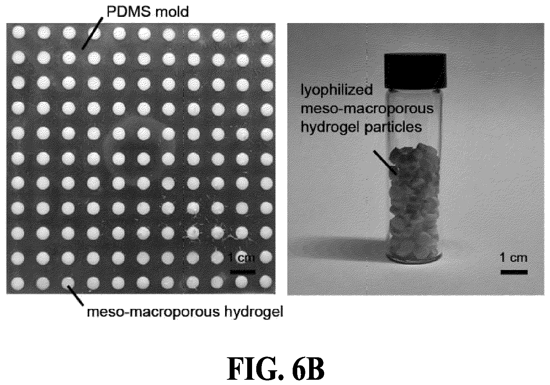

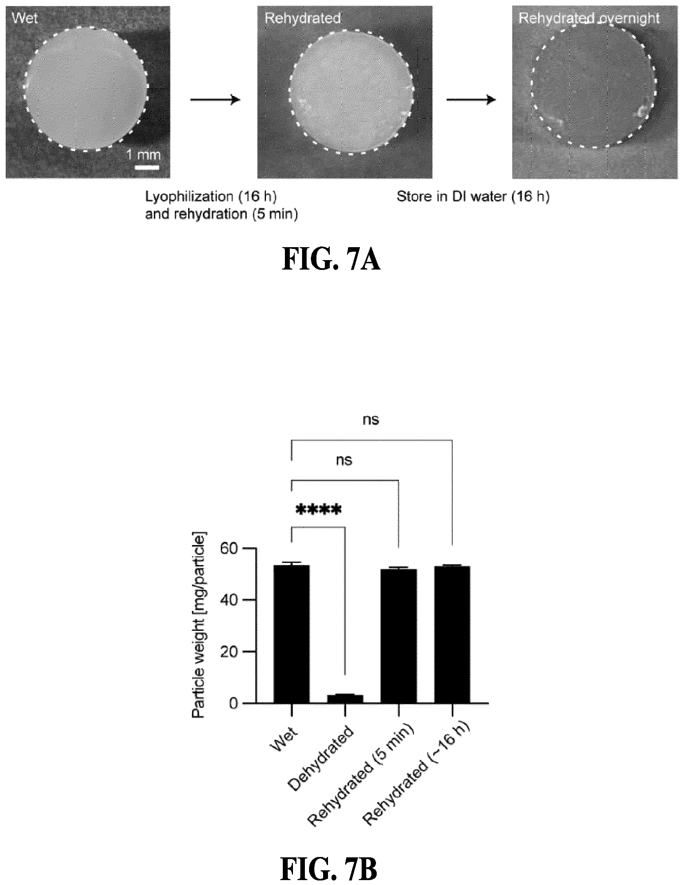

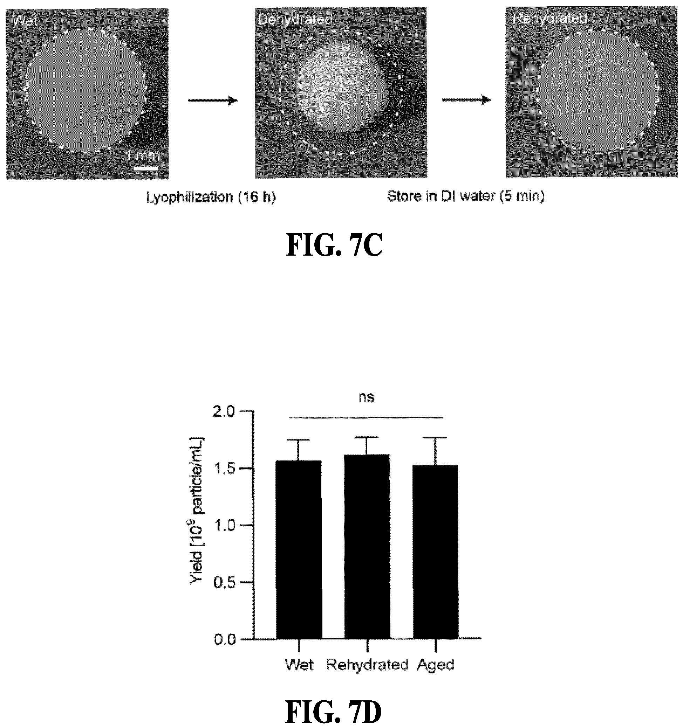

The simple preparation process described above can be easily transitioned from laboratory studies to industrial environments for low-cost, mass production, and long-term expiration date without deteriorating performance characteristics. The material cost for preparing single-tablet particles for isolating EVs from 1 mL of plasma is less than 1 cent (above Table 1). FIG. 6B shows a feasible snapshot of mass production as long as a polydimethylsiloxane (PDMS) well array and a wide-field exposure device for ultraviolet (UV, 365 nm), such as a UV chamber or a photomask aligner, may be used. In addition, the mass-produced hydrogel particles may be lyophilized and stored in a lyophilized state for a long period (e.g., 1 year or more). Moreover, the meso-macroporous hydrogel particles according to an embodiment maintained the structural integrity after dehydration and rehydration cycles. Unlike macroporous hydrogels in which excessive swelling occurred due to irreversibility of mechanical strength after dehydration, in the meso-macroporous hydrogel particles according to an embodiment, the original size and wet weight were recovered within 5 minutes in water and the size was maintained overnight without swelling (FIGS. 7A to 7C). Furthermore, to confirm the short-term and long-term stability, in-gel EV isolation was performed under three conditions of 1) immediate preparation (wet), 2) rehydration after lyophilization, and 3) 1-year aging under accelerated conditions of 94°C for 3 days. It was predicted that the increased temperature would provide an acceleration factor of 128 (2⁷) compared to storage at 24°C. In addition, it was observed that there was no statistically significant difference in EV recovery from plasma supporting the long-term stability (FIG. 7D).

### Example 5. Wide Application Of Gel-Based EV Isolation

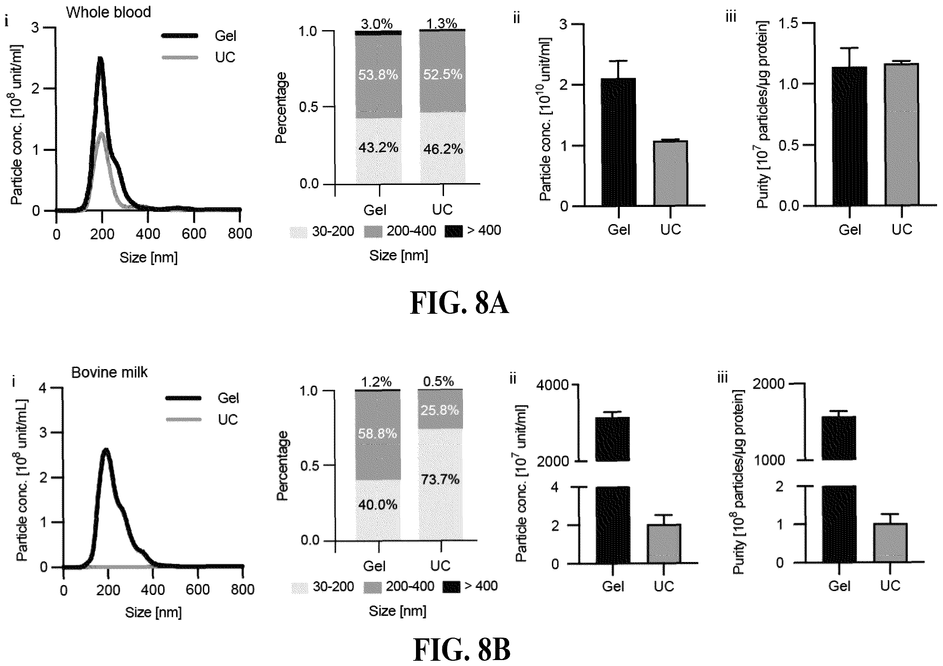

The meso-macroporous hydrogel particles provide various applications for all EV biofluids as well as human plasma. Gel-based isolation of EVs was performed from various biofluids, such as human whole blood, milk, human saliva (oral swirl), urine, and a culture medium for H1 human embryonic stem cells (H1ESC) (FIG. 8A to FIG. 8E).

First, the efficiency and purity of hydrogel-based EV isolation from whole blood were evaluated (FIG. 8A). Nanoparticle tracking analysis (NTA) bound with binning of an area under curve (AUC) at three subgroup sizes (i.e., 30 to 200, 200 to 400, and > 400 nm) showed that 97.0% of off-gel eluted nanoparticles were EVs (30 to 400 nm) and 43.2% of small EVs (sEVs; 30 to 200 nm) was included (FIG. 8A, i). These proportions were very similar to subgroups having sizes observed in ultracentrifugation (UC), which was a traditional standard for EV isolation. Furthermore, the gel-based protocol of the present disclosure significantly improved twice EV recovery (FIG. 8A, ii) without damaging the purity (FIG. 8A, iii). Considering the high clinical potential of whole blood EVs, direct isolation of EVs without a need for a preprocessing step showed the utility of the present disclosure.

Milk-derived EVs were recognized for easy obtainment of a source and broad therapeutic potential in the skin and digestive tract. However, since various non-EV impurities such as whey, fats, etc. are mixed in large quantities, more active impurity removal operation is essential before EV isolation, and as a result, EV isolation is particularly difficult and complicated compared to other biofluids, and many EVs are reported to be lost during the preprocessing process. The gel-based protocol of the present disclosure was not significantly affected by the degree of impurities in the EV-derived source, and EVs could be recovered from milk without preprocessing (impurity removal). As a result, it was confirmed that the content of impurities larger than 400 nm was 1.2%, which was not much different from 0.5% in existing methods, without an additional impurity removal process, while the yield was significantly improved. (FIG. 8B)

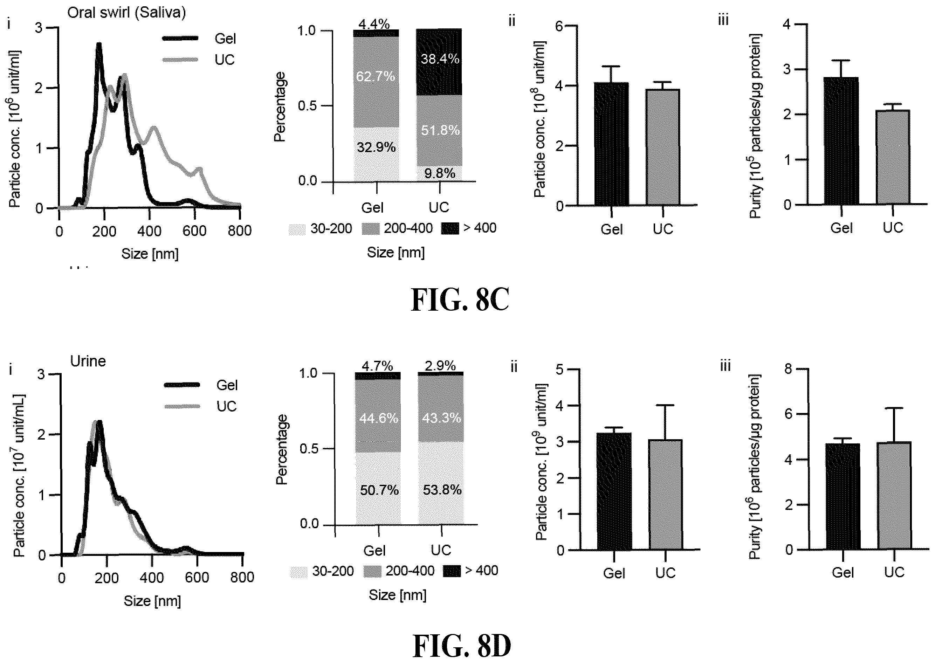

Oral swirl has recently attracted attention due to its noninvasive obtainment of clinical saliva and the potential utility of salivary EV-miRNA for disease diagnosis and prognosis. However, it has been reported that the saliva was difficult to be analyzed, including EV isolation, due to viscosity and inaccuracy that vary for person to person. It was attempted to overcome these limitations by processing the meso-macroporous hydrogel particles. Despite similar yield (FIG. 8C, ii) to slightly higher purity (FIG. 8C, iii), the off-gel recovered nanoparticles were 95.6% EV with 32.9% sEV, whereas ultracentrifugation generated 38.4% large (> 400 nm) aggregates (FIG. 8C, i), which was shown that EVs were broken by undesirable high shear stress in viscous media, and only a small fraction (9.8%) of 30 to 200 nm sEVs existed.

Urine was also an easily available body fluid with a relatively large volume (> tens mL) and is gradually suitable for *in vitro* early diagnosis. Compared with the three biofluids, meso-macroporous hydrogel particles and ultracentrifugation showed similar performance in EV isolation (FIG. 8D).

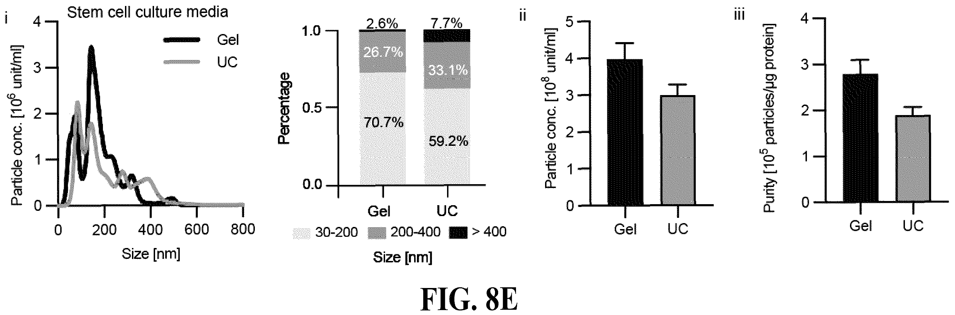

Stem cell-derived EVs isolated from the culture medium have attracted increasing attention due to therapeutic efficacy. EVs isolated from the stem cell culture medium showed slightly higher yield (FIG. 8E, ii) and purity (FIG. 8E, iii), but the size distribution of in-gel isolation showed more abundant EVs, i.e., 70.7% sEVs, with a size of 30 to 200 nm (FIG. 8E, i). These results suggest that the meso-macroporous hydrogel particles according to an embodiment may be an in-line tapping tool for monitoring differentiation lineage and degree through genetic profiling without sacrificing precious stem cells for immunostaining differentiation markers. In addition, the extended application to MSCs may be applied to refine the therapeutic aspect of EVs.

### Example 6. Evaluation Of Hydrogel-Based EV Isolation Performance

To verify the utility of the EV isolation method based on meso-macroporous hydrogel particles, several established approaches for isolating EVs from human pooled plasma (density gradient ultracentrifugation (DGUC), ultracentrifugation (UC), size exclusion chromatography (SEC), and ExoQuick (EQ)) were compared with the hydrogel-based isolation method according to an embodiment.

As shown in FIGS. 4B to 4E, the gel-based method according to an embodiment consistently showed higher EV isolation yield and/or higher purity than other existing techniques. Although the isolation yield and purity of the method according to an embodiment are slightly lower than those of SEC, the SEC is applicable to a limited range of sample types and volumes, requires sample pretreatment, and often has high isolation costs. In contrast, the gel-based method according to an embodiment was able to provide a comprehensive solution for precisely controlling the final concentration of isolated EVs in all input volumes. In particular, in new cases where the sample amount is extremely limited, such as single EV or embryonic EV characterization, gel-based methods may outperform SEC by providing a desired EV concentration.

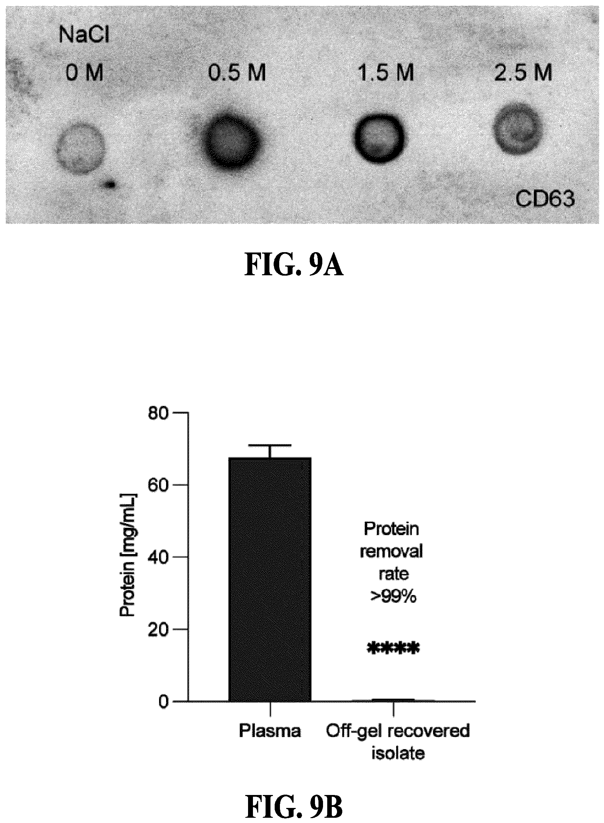

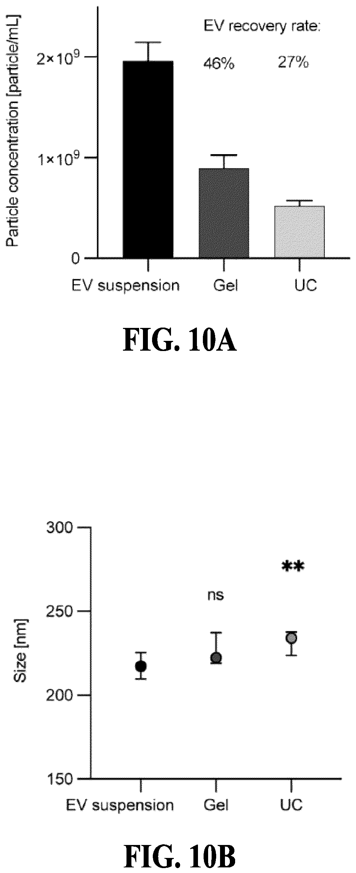

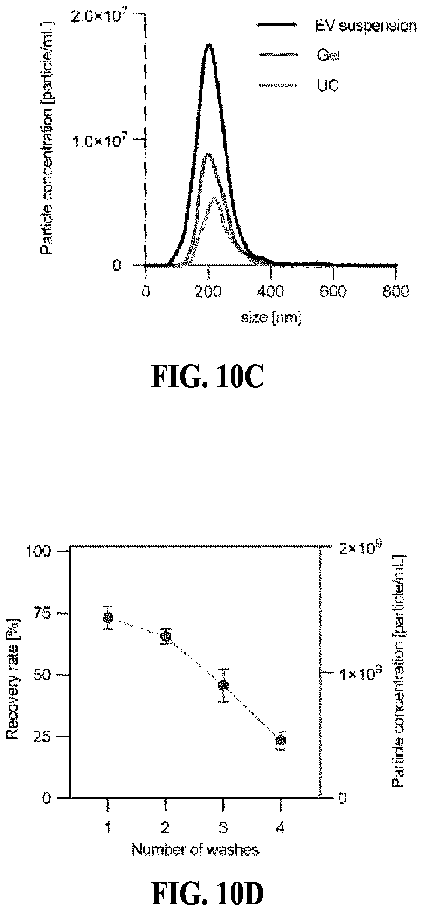

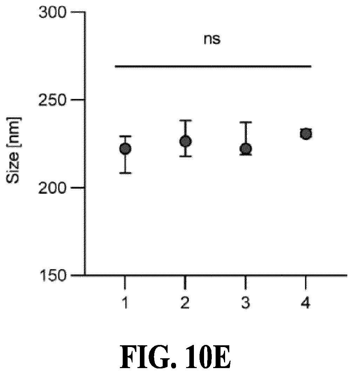

As a result of investigating the recovery rate, it was showed that 99% or more of protein impurities were removed after isolation of hydrogel-based EVs from plasma (FIG. 9B), and 46% of EVs were recovered from the exosome standard. In addition, a similar size distribution of about 220 nm was observed, whereas the UC method recovered 27% of EVs from the exosome standard with a much larger size distribution (-230 nm) due to collapse by unfavorable shear stress during ultracentrifugation (FIGS. 10A to 10C).

However, in-gel EV isolation had a critical impact on additional downstream analyses, such as multiplex detection of urinary EV-miRNA. More specifically, a clinical retrospective diagnosis of prostate cancer was successfully reconfirmed by using human urinary EVs isolated into hydrogel particles (FIG. 10D). In contrast, ultracentrifugation was unable to detect urinary EV-miRNA. Urinary EVs were isolated from 12 healthy controls and 12 prostate cancer patients, and each with 8 mL of urine was collected. Proportional profiling of two EV-miRNAs (i.e., miR-6090 and -3665) from lysates of off-gel recovered EVs consistently indicated significant differentiation between healthy controls and prostate cancer patients.

As described above, although the embodiments have been described by the restricted drawings, various modifications and variations can be applied on the basis of the embodiments by those skilled in the art. For example, even if the described techniques are performed in a different order from the described method, and/or components such as a system, a structure, a device, a circuit, and the like described above are coupled or combined in a different form from the described method, or replaced or substituted by other components or equivalents, an appropriate result can be achieved.

Therefore, other implementations, other embodiments, and equivalents to the appended claims fall within the scope of the claims to be described below.

## Claims

1. A method of producing a meso-macroporous hydrogel structure, the method comprising:
a) providing a precursor solution including 10 to 20% [v/v] of poly(ethylene glycol) diacrylate (PEGDA), 78 to 88% [v/v] of distilled water, and 2% [v/v] of a photoinitiator;
b) freezing the precursor solution at a low temperature to grow crystals;
c) forming a gelled hydrogel by photocrosslinking the frozen precursor solution with ultraviolet irradiation; and
d) thawing and washing the hydrogel at room temperature.

2. The method of claim 1, wherein ultraviolet rays of step c) have a wavelength of 365 nm.

3. The method of claim 1 or 2, wherein the ultraviolet irradiation in step c) is performed for at least 2 minutes at a UV power density of 2.5 mW/cm².

4. The method of one of claims 1 to 3, wherein PEG of step a) has a number average molecular weight (Mn) of 500 to 1,000.

5. The method of one of claims 1 to 4, wherein the photoinitiator of step a) is 2-hydroxy-2-methylpropiophenone.

6. The method of one of claims 1 to 5, wherein the freezing in step b) is freezing at -80°C for at least 10 minutes.

7. A meso-macroporous hydrogel structure produced by the method of claim 1.

8. The meso-macroporous hydrogel structure of claim 7, wherein pores of the meso-macroporous hydrogel structure have a diameter of 50 to 400 nm.

9. The meso-macroporous hydrogel structure of claim 7 or 8, wherein the meso-macroporous hydrogel structure is used for an isolation of exosomes.

10. A method of isolating exosomes using a meso-macroporous hydrogel structure, the method comprising:
a) mixing a biological sample of a subject with an exosome precipitation buffer having an NaCl concentration of 1.5 M or higher;
b) adding the meso-macroporous hydrogel structure of claim 6 to the mixture of the exosome precipitation buffer and the biological sample and precipitating exosomes inside a gel; and
c) adding the hydrogel structure in which the precipitating is completed to an exosome elution buffer having an NaCl concentration of less than 1.5 M to elute the exosomes.
